# EUROPEAN PATENT APPLICATION

(11) **EP 1 645 627 A1**
(43) Date of publication of application: **12.04.2006**
(21) Application number: 04023917.0
(22) Date of filing: 07.10.2004
(51) Int. Cl.: C12N 5/10, G01N 33/50, G01N 33/68

(54) **Quadruple transformed cell line useful for the identification of transport inhibitors**

(71) Applicant: Deutsches Krebsforschungszentrum Stiftung des öffentlichen Rechts, 69120 Heidelberg (DE)
(72) Inventor: Kopplow, Kathrin, 65307 Bad Schwalbach (DE); König, Jörg, 64347 Griesheim (DE); Keppler, Dietrich, 69221 Dossenheim (DE)
(74) Representative: Schüssler, Andrea

(57) **Abstract**

Described is a cell line containing (a) three DNA sequences encoding different uptake transporters for organic anions, preferably OATP1B1, OATP1B3 and OATP2B1, operatively linked with a promoter and (b) a DNA sequence encoding an export pump for organic anions or anionic conjugates, preferably the multidrug resistance protein 2 (MRP2), operatively linked with a promoter. Moreover, various uses of said cell line are described, preferably for the identification of transport inhibitors, e.g. drug candidates.

## Description

The present invention relates to a cell line containing (a) three DNA sequences encoding different uptake transporters for organic anions, preferably OATP1B1 (=OATP2), OATP1B3 (=OATP8) and OATP2B1 (=OATP-B), operatively linked with a promoter and (b) a DNA sequence encoding an export pump for organic anions or anionic conjugates, preferably the multidrug resistance protein 2 (MRP2, ABCC2), operatively linked with a promoter. The present invention also relates to various uses of said cell line, preferably for the identification of transport inhibitors, e.g. drug candidates.

Vectorial transport is an important function of all polarized cells contributing to detoxification and to the prevention of entry of toxins into organs. This is exemplified by kidney proximal tubule epithelia, by cells of the blood-brain barrier, by intestinal epithelia, and, last but not least, by hepatocytes. One of the major hepatocellular functions is the removal of endogenous and exogenous substances from the blood circulation and their secretion into the bile. Two transport processes play a decisive role in this vectorial transport by hepatocytes: the sinusoidal (basolateral) uptake from blood and the canalicular (apical) secretion into bile. In human hepatocytes, the sodium-independent uptake of amphiphilic organic anions is mediated by at least three transport proteins, namely by the human organic anion transporters OATP1B1 (also known as OATP2, OATP-C or LST1, symbol SLC01B1 or SLC21A6) (Abe et *al.,* J. Biol. Chem. 274 (1999), 17159-63; Hsiang et *al., J. Biol. Chem.* 274 (1999), 37161-8; König et *al.,* Am. *J. Physiol. Gastrointest. Liver Physiol.* 278 (2000), G156-64; Cui et *al., J. Biol. Chem.* 276 (2001), 9626-30), human OATP1B3 (OATP8, SLCO1B3 or SLC21A8) (König et *al., J. Biol. Chem.* 275 (2000), 23161-8; Cui et al., Mol. Pharmacol. 60 (2001), 934-43)), and human OATP2B1 (OATP-B, SLCO2B1 or SLC21A9) (Kullak-Ublick et *al., Semin. Liver Dis.* 20 (2000), 273-93). All three transporters belong to the subgroup SLCO (or 21A) of the solute carrier (SLC) superfamily. Whereas OATP2B1 is expressed also in a number of other tissues, OATP1B1 and OATP1B3 are expressed exclusively in human hepatocytes. The substrate spectrum of OATPs includes bile salts, conjugates of steroid hormones, thyroid hormones, and many other amphiphilic organic anions (Abe et *al.* (1999); Cui et *al.* (2001); Kullak-Ublick et *al.* (2000), Gastroenterology 120 (2001), 525-33). Unlike these basolateral uptake transporters which are thought to be of exchanger type, the apical export transporters identified in human hepatocytes so far are members of the ATP-binding cassette (ABC) superfamily. The export of organic anions is predominantly mediated by the bile salt export pump BSEP (ABCB11) belonging to the MDR (ABCB) subgroup of the ABC superfamily and by the multidrug resistance protein 2 (MRP2, ABCC2) belonging to the MRP (ABCC) subgroup of the ABC superfamily. While the major substrates of BSEP are bile salts like cholyl taurine and cholate, the organic anions transported by MRP2 are mainly conjugates of lipophilic substances with glutathione, glucuronate, or sulphate.

The transhepatic transport of amphiphilic organic anions has been frequently studied by use of model compounds like sulfobromophthalein (BSP) and indocyanine green (ICG) (Scharschmidt et *al., J. Clin. Invest.* 56 (1975), 1280-1292). Functional characterization of the three human OATPs identified in the hepatocyte basolateral membrane demonstrated that all three are able to mediate the uptake of BSP, with the highest affinity for OATP1B1 (Kₘ = 140 nM) and the lowest affinity (Kₘ = 3.4 *µ*M) for OATP1B3. In the hepatocyte BSP is predominantly conjugated with glutathione to yield the BSP glutathione *S-*conjugate (BSP-SG). Studies with transport-deficient mutant rats lacking functional MRP2 in the apical membrane suggested that this export pump mediates the secretion of BSP-SG into bile.

When developing or designing new pharmaceuticals one of the critical questions is to whether the candidate compounds have undesired side effects like interference with the transport of substances which are produced or occur naturally in the body, e.g. interference with the hepatic or renal transport of organic anions, as exemplified by the interference of rifampicin, rifamycin SV, or CDNB with the transcellular transport of BSP. For cost saving, it is desirable to detect such side effects of drug candidates at an early stage of development. So far, for the study of potential side effects of drug candidates animals or cell cultures were used. However these approaches exhibit a variety of disadvantages, e.g. are time and cost consuming, do not allow the high throughput screening of drug candidates etc.. Moreover, previously double-transfectants based on MDCK cells were constructed for screening candidate drugs (Cui et al., Mol. Pharmacol. 60 (2001), 934-43: OATP1B3 + MRP2); Sasaki et al. (2002), J. Biol. Chem. 277, 6497-6503: OATP1B1 + MRP2). However, the reliable identification of substrates that are transported only by one of the OATPs requires the use of three different cell lines (double-transfectants). Unfortunately, such cell lines are not available and, of course, screening procedures requiring the use of three different cell lines are cost-intensive and time-consuming.

Therefore, it is the object of the present invention to provide a means for the efficient analysis of the interference of a drug candidate with transport of substances which are produced or occur naturally in the body, particularly interference with the hepatic or renal transport of organic anions which overcomes the disadvantages of the systems presently used.

According to the invention this is achieved by the subject matters defined in the claims. A quadruple transfected cell line with defined human uptake and export transporters was established, i.e. a quadruple-transfected MDCKII cell line permanently expressing three recombinant uptake transporters for organic anions in the basolateral membrane and an ATP-dependent export pump for anionic conjugates in the apical membrane. Basolateral uptake was mediated by the human organic anion transporters OATP1B1, OATP1B3 and OATP2B1 that are the predominantly expressed transporters for organic anions in human hepatocytes and subsequent apical export by the multidrug resistance protein 2 (MRP2; ABCC2). Under physiological conditions, these transport proteins are strongly expressed in hepatocytes and contribute to the hepatobiliary elimination of organic anions. Expression and localization of OATP1B1, OATP1B3, OATP2B1 and MRP2 in MDCK cells growing on Transwell membrane inserts was demonstrated by immunoblotting and confocal laser scanning microscopy.

To conclude, the cell lines of the present invention for the first time provide a system allowing to reliably determine in a single screening experiment which drug candidates, in particular organic anions, are taken up by hepatocytes and transported into the gallbladder. Moreover, the cell lines of the present invention for the first times allows to determine interferences of different drugs not only on the level of uptake transport but also on the level of the MRP2 mediated excretion into the gallbladder. A further advantage of the cell system of the present invention vis-a-vis the presently used cells is that it in case of interference of the drug candidate with one of the transport proteins the remaining OTPs are capable of compensating the transport deficiency. Results obtained by this approach yield important information for the assessment of drug interferences and, thus, allow to make predictions for potential side effects. Finally, by use of the cell lines of the present invention an improved characterization of the substrate specificity of the apical ATP-dependent transporter MRP2 can be made which is due to the different substrate specificities of the three OATP transporters. The cell lines of the present invention are particularly useful for high throughput screening methods.

Accordingly, the present invention provides a cell line, preferably a stable cell line containing (a) three DNA sequences encoding different uptake transporters for organic anions operatively linked with a promoter and (b) a DNA sequence encoding an export pump for organic anions or anionic conjugates operatively linked with a promoter. Preferably, the uptake transporter for organic anions is expressed in the basolateral cell membrane and the export pump for organic anions or anionic conjugates is expressed in the apical cell membrane.

In certain cases, e.g., for investigating the effects of the intracellular drug metabolism on transport it might be desirable to transfect the cells of the invention with one or more additional DNA sequences encoding polyppetides the nature of which may vary according to the drug to be studied, e.g., a further transporter or metabolizing enzyme.

Preferably, for transfecting the cells the DNA sequences are present in a vector or expression vector. A person skilled in the art is familiar with examples thereof. The DNA sequences can also be contained in a recombinant virus containing appropriate expression cassettes. Suitable viruses that may be used in the present invention include baculovirus, vaccinia, sindbis virus, SV40, Sendai virus, adenovirus, an AAV virus or a parvovirus, such as MVM or H-1. The vector may also be a retrovirus, such as MoMULV, MoMuLV, HaMuSV, MuMTV, RSV or GaLV. For expression in mammals, a preferred suitable promoter is the human cytomegalovirus "immediate early promoter" (pCMV).

For generating the above described DNA sequences and for constructing expression vectors which contain said DNA sequences, it is possible to use general methods known in the art. These methods include e.g. in vitro recombination techniques, synthetic methods and in vivo recombination methods as described in Sambrook et al., Molecular Cloning, A Laboratory Manual, 2^{nd} edition (1989) Cold Spring Harbor Laboratory Press, Cold Spring Harbor, NY, for example. Methods of transfecting the cells, of phenotypically selecting transfectants and of expressing the DNA according to the invention by using the above described vectors are also known in the art.

Preferably, the cell line is a human cell line, e.g. HEK293. Particularly preferred are polarized cells, e.g. hepatocytes, kidney cells, e.g. MDCKII or HepG2.

In a further preferred embodiment of the cell line of the present invention the uptake transporters for organic anions are members of the subgroup SLCO (SLC21A) of the solute carrier (SLC) superfamily. Particularly preferred are OATP1B1, OATP1B3 and OATP2B1.

In a more preferred embodiment of the cell line of the present invention the export pump for organic anions or anionic conjugates is a member of the MDR (ABCB) subgroup or the MRP (ABCC) subgroup of the ABC superfamily. Particularly preferred are the bile salt export pump BSEP (ABCB11) or the multidrug resistance protein 2 (MRP2).

In an even more preferred of the cell line of the present invention the DNA sequence encoding an uptake transporter for organic anions and/or the DNA sequence encoding an export pump for organic anions or anionic conjugates are operatively linked with a promoter allowing high expression.

Finally, the present invention relates to various uses of the double-transfected cell line. A preferred use is the identification of a transport substrate or a transport inhibitor, particularly a drug candidate. Suitable assay formats are known to the person skilled and, e.g., described in the examples, below. A preferred assay format is high throughput screening.

### Brief description of the drawings

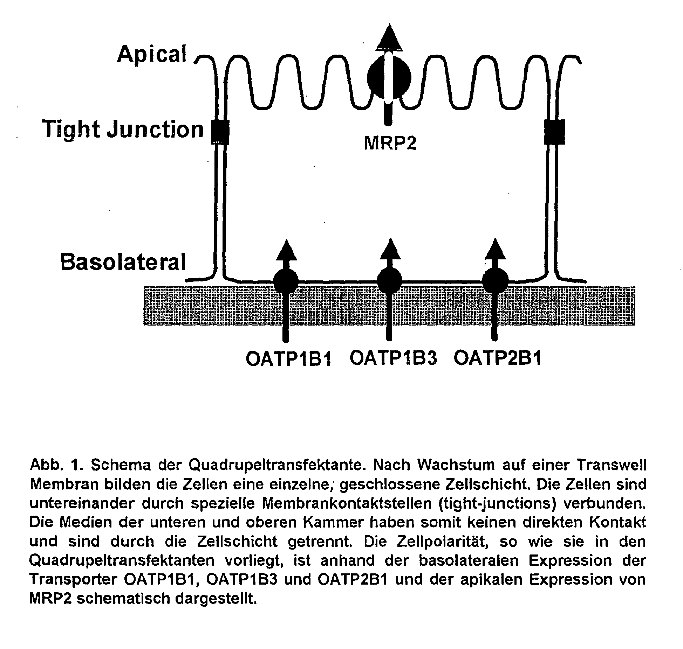

### Figure 1: Scheme of the quadruple transfectant

After proliferation on a Transwell membrane the cells constitute a single layer. The cells are intercellularly connected by particular "tight-junctions". Thus, the media of the lower and upper chamber do not have any direct contact and are separated by the cell layer. The polarity of the cells of the quadruple transfectants is schematically shown with the basolateral expression of the transporters OATP1B1, OATP1B3 and OATP2B1 and the apical expression of MRP2.

### Figure 2: Immunolocalization of recombinantly expressed transporters MRP2, OATP1B1, OATP1B3 and OATP2B1 in MDCKII cells

See Example 2, for details.

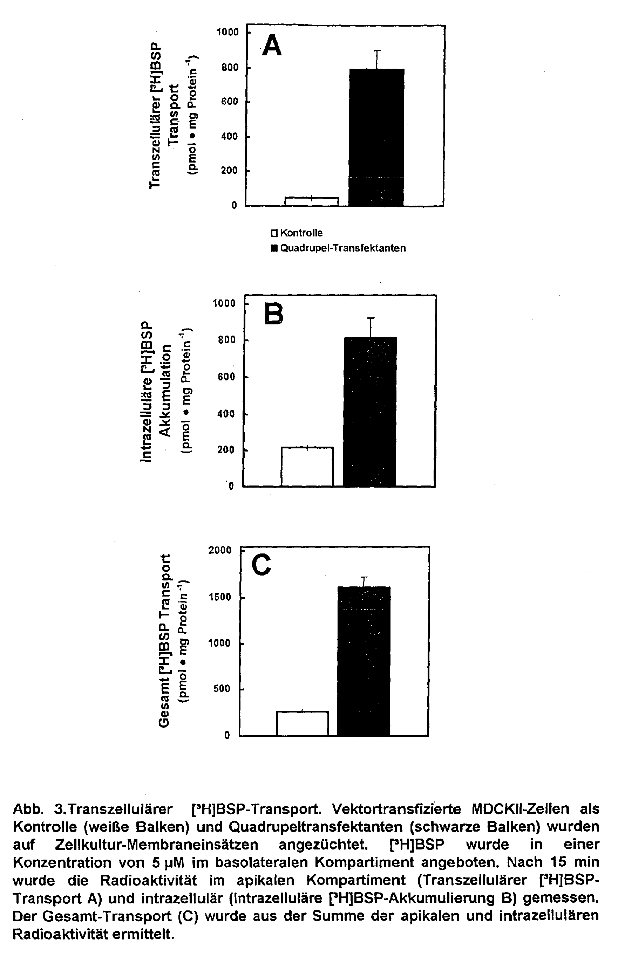

### Figure 3: Transcellular transport of the substrate [³H]BSP

See Example 3, for details.

The present invention is explained by the following examples.

### Example 1

### Materials and Methods

### (A) Chemicals

[³H]BSP (Bromosulfophthalein) (0.5 TBq/mmol) was obtained from Hartmann Analytic (Köln, Germany) by custom synthesis and non-radioactive BSP was obtained from Sigma. G418 (geneticin) sulfate was from Life Technologies (Gaithersburg, MD). Hygromycin G, Zeocin and Balsticidin was from Invitrogen (Groningen, The Netherlands). Additional non-radioactive chemicals of analytical purity were obtained from Sigma.

### (B) Construction of recombinant vectors

cDNA encoding OATP1B1 (EMBL/GenBank accession number NM_006446) was cloned as described in König et al., Am. J. Physiol. Gastrointest. Liver Physiol. 278 (2000), G156-64, and subcloned into the expression vector pcDNA3.1./Hygro(-). cDNA encoding OATP2B1 (EMBL/GenBank accession numbert NM_007256) was amplified using the primers pOATP2B1.for (5'-ACTCCAGCAGTCATGGGACC-3') and pOATP2B1.rev (5'-ACGAGTAGGATTCTCCTCACTT-3') and subcloned into the expression vector pCDNA6/V5-HisB. cDNA encoding OATP1B3 (EMBL/GenBank accession number NM_019844) was cloned as described in König et al., J. Biol. Chem. 275 (2000), 23161-8, and subcloned into the expression vector pcDNA3.1/Zeo(+). cDNA encoding MRP2 (EMBL/GenBank accession number X96395) was cloned as described in Cui et al., Mol. Pharmacol. 55 (1999), 929-37, and subcloned into the expression vector pcDNA3.1(+).

### (C) Cell Culture and Transfection

MDCKII (strain II of Madin-Darby canine kidney) cells were cultured in minimum essential medium Eagle supplemented with 10% fetal bovine serum, 100 U/ml penicillin, and 100 µg/ml streptomycin at 37°C, 95% humidity, and 5% CO₂. The MRP2-OATP1B1 double-transfectant (described in Fehrenbach et al., Naunyn Schmiedebergs Arch. Pharmacol. 385 (2003), 415-20) was transfected with the expression vector pcDNA3.1Zeo(+) containing the cDNA encoding OATP1B3 by the metafectene method (Biontex); selection was carried out with Zeocin (1 mg/ml). Then, the clone stably expressing MRP2, OATP1B1 and OATP1B3 was transfected with the expression vector pCDNA6/V5-HisB containing the cDNA encoding OATP2B1; selection was carried out with Blasticidin (10 µg/ml). Cells of the clone showing the highest expression of MRP2 and, in addition, homogeneous expression of OATP1B1, OATP1B3 and OATP2B1 were designated as quadruple transfected cells and "MDCK-MRP2-OATP1B1-OATP1B3-OATP2B1 Quadruple Transfected Cells", respectively, and used for further experiments.

### (D) Immunofluorescence analysis

The apical localization of MRP2 and the basolateral localization of OATP1B1, OATP1B3 and OATP2B1 in the quadruple transfectants was demonstrated by use of confocal laser scanning microscopy. The quadruple transfectants were grown on Transwell membrane inserts (6.5 mm diameter, 0.4 *µ*m pore size, Corning Costar, Bodenheim, Germany) for 3 days at confluency and subsequently induced with 10 mM sodium butyrate for 24 h (Cui et *al.,* 1999). Cells were fixed with 2.5% paraformaldehyde in PBS (137 mM NaCl, 2.7 mM KCl, 8.0 mM Na₂HPO₄, 1.5 mM KH₂PO₄, pH 7.4) for 20 min as described in König et al., Am. J. Physiol. Gastrointest. Liver Physiol. 278 (2000), G156-64.

Stable expression of the transporters in the quadruple transfectants was determined by use of polyclonal antisera ESL (König et al., Am. J. Physiol. Gastrointest. Liver Physiol. 278 (2000), G156-64) and SKT (König et al., J. Biol. Chem. 275 (2000), 23161-8) at a dilution of 1:50. MRP2 was determined in the quadruple transfected cells by use of the commercially available monoclonal antibody M₂III6 (Alexis). For detection of OATP2B1, a polyclonal antiserum (SPA) was prepared which was directed against the C-terminal amino acid sequence of the protein OATP2B1 (SPAVEQQLLVSGPGKKPEDSRV). For detection, the antiserum SPA was used at a dilution of 1:200.

### (E) Transport Assays

Transport assays were carried out as described in Cui et al., Mol. Pharmacol. 60 (2001), 934-43. The apical localization of MRP2 and the basolateral expression of OATP1B1, OATP1B3 and OATP2B1 in the quadruple transfectants was shown by the generation of polar proliferation of the cells after initial growing on Transwell membrane inserts (24 mm diameter, 0.4 µM pore size, Corning Costar) and by use of confocal laser scanning microscopy. The transcellular transport of substrates on the Transwell membranes was demonstrated by use radioactively labelled bromosulfophthalein (BSP). BSP is a substrate for all of the transporters of the quadruple transfectant and is transported from the basolateral compartment into the apical compartment of the Transwell membrane inserts. In addition, part of the substance accumulates intracellularly.

### Example 2

### Localization of recombinantly expressed transporters MRP2, OATP1B1, OATP1B3 and OATP2B1 in MDCKII cells

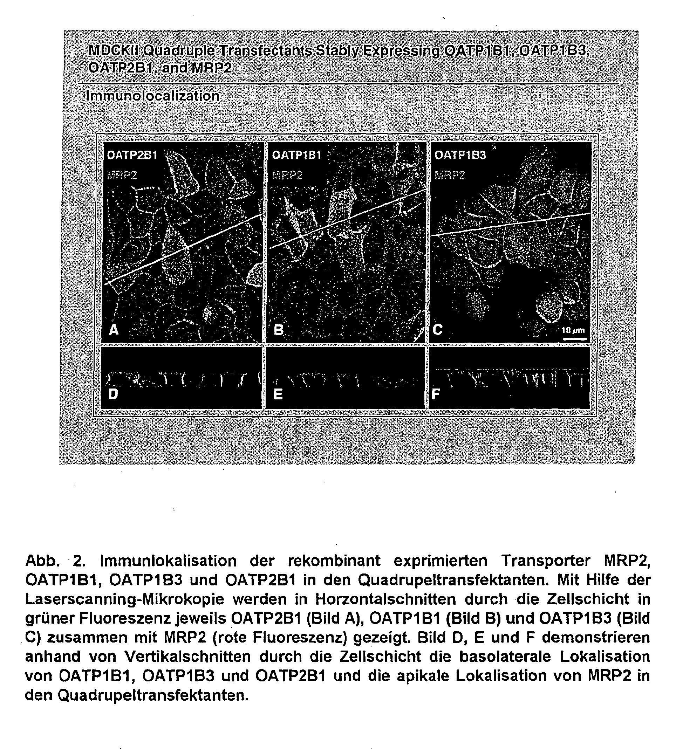

The immunolocalization of recombinantly expressed transporters MRP2, OATP1B1, OATP1B3 and OATP2B1 in quadruple transfected MDCKII cells was determined by confocal laser scanning microscopy in horizontal sections of the cells. Green fluorescence is indicative of OATP2B1 (Figure 2A), OATP1B1 (Figure 2B) and OATP1B3 (Figure 2C), red fluorescence in Figures 2A-C is indicative of MRP2. Figures 2 D, E and F are vertical sections of the cells showing the basolateral localization of OATP1B1, OATP1B3 and OATP2B1 as well as the apical localization of MRP2 within the quadruple transfectants.

### Example 3

### Transcellular Transport of [³H]BSP mediated by OATP1B1, OATP1B3, OATP2B1 and MRP2

The function of human OATP1B1, OATP1B3, OATP2B1 and MRP2 in the quadruple-transfected cells was studied by measurement of the transcellular transport of the organic anion [³H]BSP. Thus, polarized MDCKII cells grown on Transwell membrane inserts were incubated with [³H]BSP at a concentration of 5 µM in the basolateral compartments. After 15 min., the radioactivity accumulated in the apical compartment and intracellularly was measured.

## Claims

1. A cell line containing (a) three DNA sequences encoding different uptake transporters for organic anions operatively linked with a promoter and (b) a DNA sequence encoding an export pump for organic anions or anionic conjugates operatively linked with a promoter.

2. The cell line of claim 1 containing at least one further heterologous DNA sequence.

3. The cell line of claim 1 or 2 which is a canine or human cell line and the DNA sequences of (a) and/or (b) are human.

4. The cell line of any one of claims 1 to 3 which is a kidney cell line.

5. The cell line of any one of claims 1 to 4 wherein the uptake transporters for organic anions are members of the subgroup SLCO (21A) or 22A of the solute carrier (SLC) superfamily.

6. The cell line of claim 5, wherein the uptake transporters for organic anions are OATP1B1, OATP1B3 and OATP2B1.

7. The cell line of any one of claims 1 to 6 wherein the export pump for organic anions or anionic conjugates is a member of the MDR (ABCB) subgroup or the MRP (ABCC) subgroup of the ABC superfamily.

8. The cell line of claim 7, wherein the export pump for organic anions or anionic conjugates is the bile salt export pump BSEP (ABCB11) or the multidrug resistance protein 2 (MRP2; ABCC2).

9. The cell line of any one of claims 1 to 8 wherein the DNA sequences encoding uptake transporters for organic anions and/or the DNA sequence encoding an export pump for organic anions or anionic conjugates are operatively linked with a promoter allowing high expression.

10. Use of a cell line according to any one of claims 1 to 9 for the identification of a transport substrate or a transport inhibitor.

11. Use according to claim 10 wherein the transport inhibitor is a drug candidate.

12. Use according to claim 10 or 11 wherein the identification of a transport substrate or a transport inhibitor is carried out as high throughput screening.
